# EUROPEAN PATENT SPECIFICATION

(11) **EP 1 587 539 B1**
(45) Date of publication and mention of the grant of the patent: **04.08.2010**
(21) Application number: 03779776.8
(22) Date of filing: 30.12.2003
(51) Int. Cl.: A61K 39/395, A61K 38/47, C12N 9/36, A61P 31/00

(54) **ANTIMICROBIAL COMPOSITION FOR LOCAL USE ON MUCOSAL MEMBRANES AND SKIN**
ANTIMIKROBIELLE ZUSAMMENSETZUNG ZUR LOKALEN ANWENDUNG AUF DER SCHLEIMHAUT UND DER HAUT
COMPOSITION ANTIMICROBIENNE A UTILISATION LOCALE SUR DES MEMBRANES MUCOSALES ET SUR LA PEAU

(30) Priority: 02.01.2003 DK 200301858
(43) Date of publication of application: 26.10.2005
(73) Proprietor: Pedersen Medical A/S, 7100 Vejle (DK)
(72) Inventor: PEDERSEN, Jens, Richard, DK-7100 Vejle (DK); PEDERSEN, Torben, Richard, DK-7120 Vejle Oest (DK)
(74) Representative: Høiberg A/S
(86) International application number: PCT/DK2003/000940
(87) International publication number: WO 2004/060397

(56) References cited:
- EP-A- 1 068 871
- FR-A- 2 573 627
- GB-A- 2 229 725
- RU-C- 2 070 889
- US-A- 4 734 279
- DOLGUSHIN II ET AL: "The differentiated correction of the local immunity of the reproductive tract by using lysozyme and immunoglobulins i pregnant women with a genital infection " ZH MIKROBIOL EPIDEMIOL IMMUNOBIOL, vol. 4, 1997, pages 111-115, XP002280420
- DATABASE WPI Section Ch, Week 200103 Derwent Publications Ltd., London, GB; Class B04, AN 2001-019312 XP002281415 & JP 2000 270858 A (TAIYO KAGAKU KK), 3 October 2000 (2000-10-03)
- JANEWAY, CHARLES A. ET AL: "IMMUNO BIOLOGY, The Immune system in health and disease" 1999 , ELSEVIER SCIENCE LTD/GARLAND PUBLISHING , LONDON XP002280421 Fourth Edition page 101 -page 103; figure 3.21
- JACQUES BAENZIGER ET AL: "Structure of the Carbohydrate Units of IgE Immunoglobulin" THE JOURNAL OF BIOLOGICAL CHEMISTRY, vol. 249, no. 6, 25 March 1974 (1974-03-25), pages 1889-1896, XP002282069

## Description

### Field of invention

The present invention relates to an antimicrobial composition comprising lysozyme and glycosylated immunoglobulins. The present invention further relates to the method of preparation of said composition as well as use of the composition for the treatment and/or prevention of infections.

### Background of invention

Infections of the mucosal membranes and skin infections are caused by both Gram positive and Gram negative bacteria.

Antimicrobial agents are used for local use on mucosal membranes and for local use of lesions to the skin. The purpose of a local treatment may be to supplement a systemic treatment by local use in suitable cases where adequate concentrations of the antimicrobial agent cannot be achieved in local foci of infection by systemic treatment alone, e.g. in the treatment of paronychion.

Local treatment using antibiotics in the form of a cream or an ointment is also conducted in case of superficial infections. By choosing such a treatment instead of a systemic treatment with antibiotics a number of side-effects that may be associated with systemic treatment can be avoided.

Furthermore, a number of diverse disinfectants are used in the treatment of infected lesions to the skin. Both groups of antimicrobial agents for local use, the group of antibiotics and the group of disinfectants, suffer from substantial disadvantages.

### Antibiotics

By systemic treatment using antibiotics a risk of sensitization of the patient is always present but such a risk is substantially increased by local use of the antibiotic in question. For this reason one is often reluctant in cases of trivial infections of mucosal membranes and skin to locally use antibiotics in form of creams or ointments as sensitization caused by one or more antibiotics will decrease the possibility of treating a patient should a serious infection occur later in life. Furthermore, local treatment using antibiotic implicates an increased risk of the development and selection of resistant bacteria, in this respect particularly long-term treatment of skin infections is alarming.

### Disinfectants

Disinfectants for the treatment of localised or superficial infections are collectively to be recognized as general cell damaging agents. The most widely used disinfectants include iodophor, chlorhexidine, quaternary ammonium compounds, hydrogen peroxide and acids.

### Lysozyme

In 1922 the bacteriologist Alexander Flemming discovered a hitherto unknown enzyme which he termed lysozyme due to its ability to lyse a number of different bacteria.

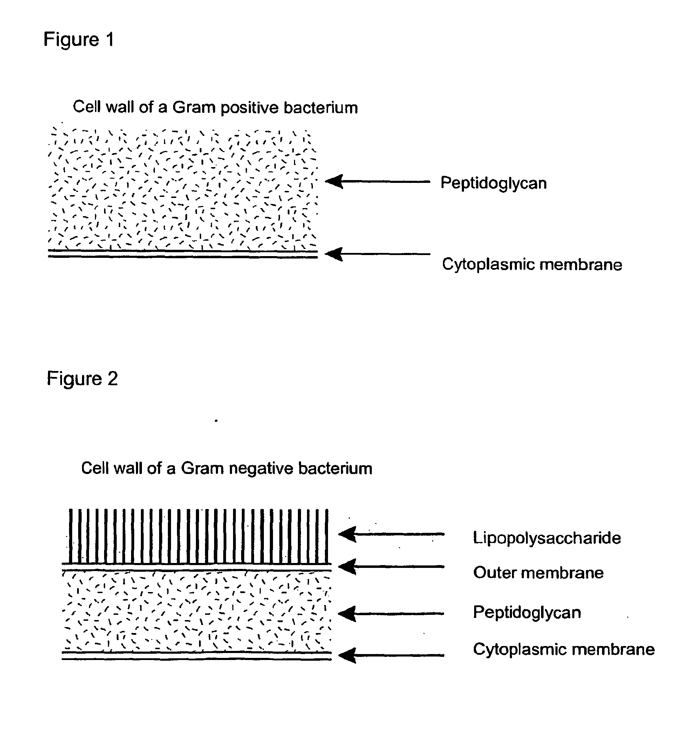

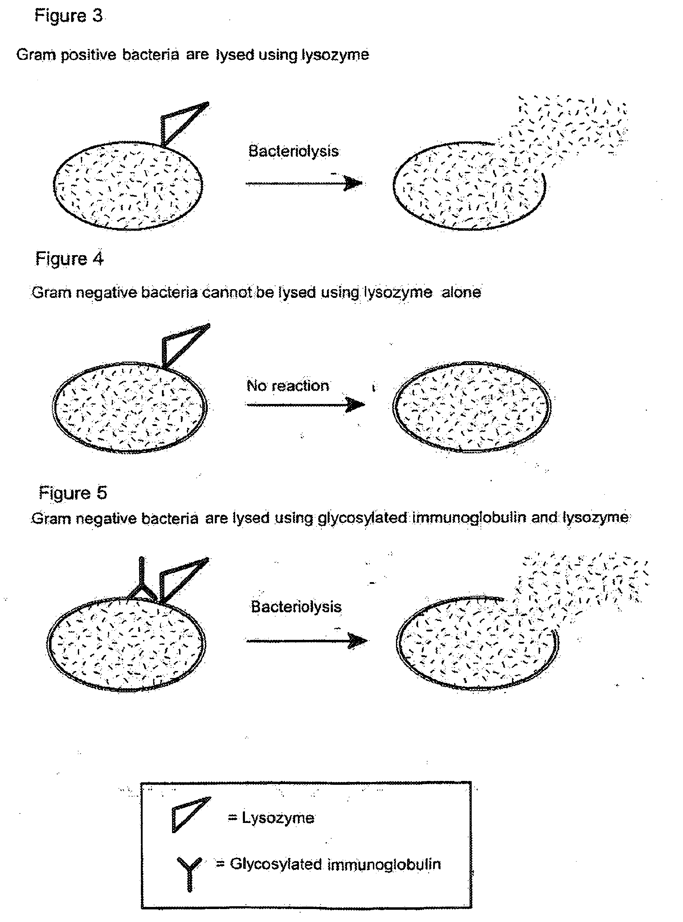

Lysozyme is present in a number of biological fluids such as egg white, milk, blood and tears. Alexander Flemming had great expectations for lysozyme as a therapeutic for treatment of infectious diseases but soon it became clear that no effect of lysozyme against the most common pathogenic bacteria could be detected The fact that lysozyme is functional against some bacteria and not against other types of bacteria is due to differences in the outer membrane of bacteria. It is well known in the art that Gram positive bacteria are lyzed in the presence of lysozyme whereas Gram negative bacteria are not affected by lysozyme. The reason for this is that the outer layer of the cell membrane of Gram positive bacteria consists of peptidoglycan (Fig. 1) which is readily degraded by lysozyme which causes the bacteria to die (Fig. 3).

The situation for Gram negative bacteria is different from that of Gram positive bacteria. In Gram negative bacteria the outer layer of the cell membrane consists of lipopolysaccharide and only after that a layer of peptidoglycan (Fig. 2). As lysozyme is not able to cleave the layer of lipopolysachharide (Fig. 4) it means that this outer layer has to be removed or to be perforated before the lysozyme can destroy Gram negative bacteria.

Through the years a number of efforts have been made in order to solve this problem without achieving satisfying results. One has tried to combine lysozyme and EDTA, polysorbate, potassium sorbate amongst other but none of these methods have been suitable for the design of a composition for use on mucosal membranes and skin, moreover, a general effect on the wide range of Gram negative bacteria has not been achieved.

### Immunoglobulins (gammaglobulins, antibodies)

In 1890 Emil von Behring published a scientific paper on the work on treatment of tetanus using antitoxin. Subsequent studies have shown that antitoxin was immunoglobulins which became the starting point for passive immunotherapy in which immunoglobulins were prophylactically injected intramuscularly or subcutaneously against for example botulism, diphtheria, adder bite and snake bite. The immunoglobulins all derived from immunized animals and were directed against toxins. Furthermore,immunoglobulins of human origin are applied in the prophylaxis of tetanus and hepatitis B.

It is well known that immunoglobulins play a role in the control of microbial infections. First, specific immunoglobulins bind to the surface of the bacteria whereby opsonization is possible which renders the bacterium in question more attractive to the phagocytes. It is furthermore well known that immunoglobulins on their own are not able to kill bacteria. It is necessary that an intact complement system is present which is activated by the immunoglobulins in order to kill bacteria. The fact that immunoglubulins are dependent on complement or phagocytes in the battle against bacteria has the effect that immunoglobulins are only seen to be capable of having an effect when they are injected whereby the immunoglobulins are mixed with serum and tissue fluid in which plenty of complement is present. This notion is supported by the fact that immunoglobulins do not have any effect against bacteria under in vitro conditions.

Compositions are described for local use comprising among other things immunoglobulins and lysozyme (EP 1068071 and US 4734279). These compositions undoubtedly have an effect against Gram positive bacteria due to their contents of lysozyme whereas Gram negative bacteria will not be affected by lysozyme nor lysozyme in combination with native immunoglobulins for local use. It is common knowledge that bacteria are equipped with proteolytic enzymes that are able to degrade immunoglobulins. Some bacterial enzymes have IgA1 as their specific substrate, the socalled post-proline endopeptidases. These measures are described in detail by Mogens Killian et al. in APMIS 104: 321-338, 1996. The IgG molecule that is not present in mucosal membranes is very sensitive to all sorts of proteases and it is inactivated after few minutes in most suspensions of bacteria. Thus, repeated experiments in vitro have also shown lack of effect on Gram negative bacteria using the combination of lysozyme and native immunoglobulins. Treatment of pregnant women suffering from genital infection by the use of lysozyme and native immunoglobulins has been described (Dolgushin, I.I et al (1997) ZH Mikrobiol. Epidemiol. Immunobiol. 4: 111-115), thus not disclosing synthetically glycosylated immunoglobulins. In addition, methods for enhancing the effect of antibodies by adding enzymes such as lysozyme has been desribed (GB-A-2229725, FR-A-2573627). These documents do not disclose the use of glycosylated immunoglobulins.

### Summary of invention

The present invention relates to an antimicrobial composition, preferably for local use on mucosal membranes and skin comprising a combination of lysozyme and glycosylated immunoglobulins, wherein the immunoglobulins have affinity to Gram negative bacteria.

Accordingly, the present invention relates to an antimicrobial composition comprising lysozyme and glycosylated immunoglobulins as defined in claim 1.

In another aspect the invention relates to an antimicrobial composition, comprising lysozyme conjugated to a monosaccharide, in particular mannose as defined in claims 17-18.

Lysozyme is able to lyse Gram positive bacteria by degrading peptidoglycan of the bacterial cell wall (Fig. 3). The peptidoglycan of Gram negative bacteria is protected against the action of lysozyme by a layer of lipopolysaccharide (Fig. 4). When glycosylated immunoglobulins resistant to proteases bind to the cell wall of the Gram negative bacteria the surface of the lipopolysaccharide layer is altered in such a manner that lysozyme is able to degrade the underlying peptidoglycan layer, resulting in bacteriolysis (Fig. 5).

In a further aspect the invention relates to the preparation of the antimicrobial composition described above, comprising the steps of
a) obtaining immunoglobulins
b) glycosylating the immunoglobulins
c) obtaining native or conjugated lysozyme
d) mixing the glycosylated immunoglobulins and the lysozyme, and optionally adding additives, thereby obtaining the antimicrobial composition.

Or
comprising the steps of
a) obtaining the lysozyme
b) conjugating the lysozyme, and optionally adding additives, thereby obtaining the antimicrobial composition.

Furthermore, the invention relates to the use of lysozymes and glycosylated immunoglobulins for the preparation of an antimicrobial composition as defined above. In particular the composition is useful for preventing and treating infections, and accordingly, the invention also relates to the use of lysozymes and glycosylated immunoglobulins for the preparation of an antimicrobial composition as defined above for the prophylaxis and/or treatment of an infection.

The invention also relates to the use of a conjugated lysozyme for the preparation of an antimicrobial composition as defined above. In particular the composition is useful for preventing and treating infections, and accordingly the invention also relates to the use of a conjugated lysozyme for the preparation of an antimicrobial composition as defined above for the prophylaxis and/or treatment of an infection.

### Description of Drawings

Figure 1:
   Schematic drawing of the cell wall of a Gram positive bacterium.
Figure 2:
   Schematic drawing of the cell wall of a Gram negative bacterium.
Figure 3:
   Diagram showing the lysis of a Gram positive bacterium using lysozyme.
Figure 4:
   Diagram illustrating that Gram negative bacteria are not affected by lysozyme alone.
Figure 5:
   Diagram showing the lysis of a Gram negative bacterium using glycosylated immunoglobulin and lysozyme according to the invention.

### Detailed description of the invention

### Definitions

Agglutination is a clumping of bacteria when held together by antibodies.

Antigen determinant is a surface feature of a microorganism or macromolecule, such as a glycoprotein, that elicits an immune response.

Antigen is any foreign substance, such as a virus, bacterium, or protein that elicits an immune response by stimulating the production of antibodies. An antigen is a substance that stimulates the production or mobilization of antibodies. An antigen can be a foreign protein, toxin, bacteria, or other substance.

FC fragment is the constant region on an immunoglobulin molecule. This area is exactly the same on all antibodies. The region is found on the heavy chains and is not involved in binding antigens.

Glycosylation is understood to be the process of adding sugar units such as in the addition of monosaccharides or polysaccharides to proteins.

Gram negative bacteria are bacteria whose cell wall stains pink (negative) in Gram stain. The cell wall of a gram-negative bacterium contains relatively little peptidoglycan but contains an outer membrane composed of lipopolysaccharide (LPS), lipoprotein, and other complex macromolecules.

Gram positive bacteria are bacteria whose cell wall stains purple (positive) in Gram stain. The cell wall of a Gram positive bacterium consists primarily of peptidoglycan and lacks the outer membrane of Gram negative cells.

Heavy chains are the heavier of the two types of polypeptide chains that are found in immunoglobulin and antibody molecules.

IgA is the principal antibody of the membranes of the respiratory and gastrointestinal tracts.

IgA dimer is a form of IgA in which two IgA molecules are covalently linked to a secretory component.

IgG is the main class of immunogloulins produced towards the end of a primary immune response and in a secondary responser. IgG is the main antibody defence against bacteria.

IgM is a class of immunoglobulins involved in fighting infection of the blood by attacking antigens often antigens present on bacteria. IgM triggers the production of IgG.

IgY is a class of immunoglobulins isolated from the egg yolks of hens.

Immunoglobulins are antibodies. Antibodies are found in blood and tissue fluids produced by cells of the immune system to bind to substances in the body that are recognized as foreign antigens. Immunoglobulins sometimes bind to antigens that are not necessarily a threat to health. In the text the term 'immunoglobulin' is identical to the term 'antibody'.

Monoclonal antibodies are immunoglobulin molecules of single- epitope specificity that are secreted by a clone of B cells.

Mucosal membranes are moist tissue that lines some organs and body cavities (such as nose, mouth, lungs, vagina and gastrointestinal tract) and secretes mucous (a thick fluid).

Peptidoglycan is a polymer of amino sugars (mainly N-acetylglucosamine and N-acetylmuramic acid).

Polyclonal antibodies are a mixture of immunoglobulins secreted against a specific antigen, each recognizing a different epitope.

The object of the present invention is to provide an antimicrobial composition for local use without the side-effects and the disadvantages involved when using antibiotics and disinfectants.

According to the invention this is achieved by a composition comprising a combination of lysozyme and glycosylated immunoglobulin.

The advantage of the present invention is that it has solved the problem of lysozyme penetrating the outer lipopolysaccharid membrane on Gram negative bacteria and thus created a possibility of degrading the peptidoglycan membrane resulting in bacteriolysis (Fig. 5).

The invention is based on in vitro experiments using Gram negative bacteria and glycosylated immunoglobulins directed against antigens present on the surface of the bacteria.

Upon attachment of a glycosylated immunoglobulin on the surface antigen of the bacterial wall it was shown that the bacteria could be killed using lysozyme.

The possible explanation of this phenomenon is that the glycosylated immunoglobulin molecule (the antibody) binds to the surface antigen determinant of the bacterial wall whereby the characteristics of the surface of the bacterial wall is altered in such a manner that lysozomal activity on the underlying layer of peptidoglycan is possible.

In one aspect of the invention the composition comprises a combination of lysozyme and glycosylated immunoglobulins.

In one embodiment of the invention the glycosylated immunoglobulins originate from biological fluids such as milk, whey, blood, plasma, or serum. It is desirable that the glycosylated immunoglobulins derive from milk. It is further desirable that the glycosylated immunoglobulins derive from colostrum. Moreover, it is preferred that the glycosylated immunoglobulins derive from a combination of milk and colostrum. It is further desirable that the immunoglobulins derive from egg yolks.
In a second embodiment the glycosylated immunoglobulins derive as a result of genetic engineering as recombinant proteins. Furthermore, glycosylated immunoglobulins are synthesized chemically. According to the invention the term 'immunoglobulins' covers fragments or parts of immunoglobulins that actively recognizes an antigen.

It is preferred that the used immunoglobulins remain functional for a prolonged time, also after administration. Thus, it is preferred that the immunoglobulins are intact and resistant towards bacterial proteases. This is achieved by glycosylating immunoglobulins, for example by glycosylating the FC fragment of the immunoglobulins. An example is wherein 10 g pooled immunoglobulins 77,0% IgG, 7,6% IgA and 15,4% IgM are dissolved in 25 ml 1M solution of glucose and incubated at 45°C. During the incubation the glucose will establish covalent bonds particularly to the Fc fragment of the IgG molecule which can be tested by demonstrating the loss of ability to bind complement. However, the ability of the glycosylated immunoglobulins to agglutinate is unchanged. An analogous glycosylation has been observed in vivo in insufficiently insulin-regulated patients suffering from diabetes. A glycosylated immunoglobulin is extremely resistant to pancreatic proteases as well as bacterial proteases.

In one embodiment of the invention the immunoglobulins are glycosylated at preferably the FC fragment of the immunoglobulin molecule by the establishment of covalent bonds between the FC fragment and the sugar moiety. In a particular embodiment of the invention the moiety is a monosaccharide. It is particularly preferred that the moieties are selected from glucose, galactose, fructose, mannose and talose.

It is preferred that the glycosylated immunoglobulins have half-lives that are longer than non-glycosylated immunoglobulins directed against an identical antigen assessed in vitro and/or in vivo, such as in the range of 1.1 times longer half-lives to 50 times longer half-lives, such as at least 1.1 times longer, such as at least 2 times longer, such as at least 3 times longer, such as at least 4 times longer, such as at least 5 times longer, such as at least 10 times longer, such as at least 15 times longer, such as at least 20 times longer, such as at least 25 times longer, such as at least 30 times longer, such as at least 35 times longer, such as at least 40 times longer, such as at least 45 times longer, such as at least 50 times longer.

The half-lives of the glycosylated immunoglobulins are increased due to glycosylation whereby the resistance to proteases is increased. The proteases are selected from the groups of proteases of bacterial origin, pancreatic enzymes, proteolytic enzymes originating from fruits and proteolytic enzymes found in gastric juice. Proteases of bacterial origin include bacterial post-proline endopeptidases. Proteolytic enzymes are selected from the types of papain, bromelain, trypsin and pepsin.

It is desirable that the half-live is increased by resistance to at least one protease, such as at least two proteases, such as at least three proteases, such as at least four proteases, such as at least five proteases, such as at least 10 proteases. Moreover, the half-lives of the glycosylated immunoglobulins are increased due to glycosylation whereby the resistance to acids is improved. Acids are such acids as those found in gastric juice, in particular hydrochloric acid at pH between1-3.

In one embodiment of the invention the glycosylated immunoglobulins are of polyclonal origin. In another embodiment of the invention the glycosylated immunoglobulins are of monoclonal origin. In a particular embodiment the the immunoglobulins are a combination of both monoclonal and polyclonal origin. The glycosylated immunoglobulins may be derived from immunized animals. By immunized animals is meant animals that actively have been immunized to produce antibodies against a particular antigen. Furthermore, the glycosyated immunoglobulins may derive from animal that have not actively been made to produce antibodies against a particular antigen but naturally have produced antibodies against the particular antigen. In one embodiment the glycosylated immunoglobulins derive from mammals. In a particular embodiment the glycosylated immunoglobulins derive from animals such as a rabbit, a goat, a cow, or a hamster.

At least a part of said glycosylated immunoglobulins have affinity to antigen determinants present on the surface of Gram negative bacteria. The system according to the invention is particular useful for the treatment of infections with Gram negative bacteria, such as Gram negative rods and Gram negative cocci. Of course the invention may also be used for infections with more than one microbe, such as combinations of Gram negative rods and cocci. The aerobic cocci that belong to the Gram negative bacteria are the genus including the species *N*. *meningitidis* and *N*. *gonorrhoeae.* The Gram negative rods include the Enterobacteriaceae family to which belong the genus Eschericia coli, the genus Shigella including the Serotype A- *S*. *dysenteriae*, Serotype B- *S*. *flexneri,* Serotype C- *S. boydii* Serotype D- *S*. *sonnei*, the geus Edwardsiella (e.g. *Edwardsiella tarda*), the genus salmonella (e.g. *S*. *typhimurium* and *S*. *enteritidis*), the genus citrobacter (e.g. *C. diversus, C. freundii*), the genus klebsiella (e.g. *Klebsiella pneumoniae*), the genus enterobacter (e.g. *E. aerogenes* and *E. cloacae*), the genus serratia (e.g. *Serratia marcescens*), the genus proteus (e.g. *P*. *mirabilis* and *P*. *vulgaris*)*,* the genus Morganella (e.g. *Morganella morganii*), the genus providencia (e.g. *P*. *alcalifaciens*, the genus yersinia (e.g. *Y*. *enterocolitica* and *Y*. *pestis*). The pleiomorhic Gram negative rods include the genus Haemophilus (e.g. *H*. *influenzae*, *H*. *aegyptius, H. ducreyi*), the genus Bordetella (e.g. *B*. *pertussis*, *B*. *parapertussis*), the genus Brucella (e. g. *B. abortus, B. suis, B. melitensis, B. canis*), the genus Pasteurella (e.g. *P*. *multocida*), the genus Legionella (e.g. *L. pneumophila* and *L. micdadei*)*.* The group of miscellaneous Gram negative rods include the genera vibrio (e.g. *V. chalerae, V. parahaemolyticus, V. vulnificus*). Campylobacter (e.g. *C. jejuni*), Helicobacter (e.g. *Helicobacter pylori*). Other Gram negative bacteria include the genera Pseudomonas (e.g *P*. *aeruginosa*, *B*. *cepacia*), Acinetobacter (e.g *A. baumannii, A. lwoffi*), Flavobacterium (e.g *F*. *meningosepticum*). Other Gram negative bacteria include the genera Gardnerella (e.g. G. *vaginalis*), and Chlamydia (e.g. C. *psittaci,* C. *trachomatis*).

The amount of glycosylated immunoglobulins is preferably in the range of 0,1 to 90% by weight of the composition, such as at most 80% by weight of the composition, such as at most 70% by weight of the composition, such as at most 60% by weight of the composition, such as at most 50% by weight of the composition, such as at most 40% by weight of the composition, such as at most 30% by weight of the composition, such as at most 20% by weight of the composition, such as at most 10% by weight of the composition, such as at most 9% by weight of the composition, such as at most 6% by weight of the composition, such as at most 7% by weight of the composition, such as at most 6% by weight of the composition, such as at most 5% by weight of the composition, such as at most 4% by weight of the composition, such as at most 3% by weight of the composition, such as at most 2% by weight of the composition, such as at most 1.5% by weight of the composition, such as at most 1 % by weight of the composition, such as at most 0.75% by weight of the composition, such as at most 0.5% by weight of the composition, such as at most 0.4% by weight of the composition. In another preferred embodiment of the invention the amount of glycosylated immunoglobulins is at least 0.1 % by weight of the composition, such as at least 0.2% by weight of the composition, such as at least 0.3% by weight of the composition, such as at least 0.375% by weight of the composition, such as at least 0.4% by weight of the composition. Preferably the amount of glycosylated immunoglobulins is in the range of 0.4% to 1.2% by weight of the composition, in particular the amount of glycosylated immunoglobulins is 0.4% or 1.2% by weight of the composition.

In a further embodiment of the invention the glycosylated immunoglobulins are of the classes IgG, IgA, IgM, dimer IgA and/or IgY. Preferably the IgG class of the pooled immunoglobulins are in the range of 50% to 90% by weight of the immunoglobulins, such as at least 55% by weight of the immunoglobulins, such as at least 60% by weight of the immunoglobulins, such as at least 65% by weight of the immunoglobulins, such as at least 70% by weight of the immunoglobulins, such as at least 72.5% by weight of the immunoglobulins, such as at least 75% by weight of the immunoglobulins, such as 77% by weight of the immunoglobulins, such as at most 90% by weight of the immunoglobulins,, such as at most 85% by weight of the immunoglobulins, such as at most 82.5% by weight of the immunoglobulins, such as at most 80% by weight of the immunoglobulins, such as at most 77% by weight of the immunoglobulins. Preferably the IgG class constitutes about 77 % by weight of the immunoglobulins.

It is desirable that the IgA class contents of the pooled immunoglobulins is in the range of 1% to 20% by weight of the immunoglobulins, such as at least 2% by weight of the immunoglobulins, such as at least 3% by weight of the immunoglobulins, such as at least 4% by weight of the immunoglobulins, such as at least 5% by weight of the immunoglobulins, such as at least 6% by weight of the immunoglobulins, such as at least 7% by weight of the immunoglobulins, such as at least 7.5%. Preferably the IgA class content of the pooled immunoglobulins is about 7.6% by weight of the immunoglobulins.

The IgM class contents of the pooled immunoglobulins is preferably in the range of 5% to 30% by weight of the immunoglobulins, such as at least 5% by weight of the immunoglobulins, such as at least 6% by weight of the immunoglobulins, such as at least 7% by weight of the immunoglobulins, such as at least 8% by weight of the immunoglobulins, such as at least 9% by weight of the immunoglobulins, such as at least 10% by weight of the immunoglobulins, such as at least 11%, such as at least 12% by weight of the immunoglobulins, such as at least 13% by weight of the immunoglobulins, such as at least 14% by weight of the immunoglobulins, such as at least 14.5% by weight of the immunoglobulins, such as at least 15% by weight of the immunoglobulins. Preferably the IgM class content of the pooled immunoglobulins is about 15.4% by weight of the immunoglobulins of the composition.

In yet another embodiment of the invention the glycosylated immunoglobulins are only of the IgG class, whereby extracted glycosylated IgG is used as the only immunoglobulin in the antimicrobial composition. Furthermore, glycosylated IgG in a pure form can be mixed with pooled immunoglobulins in the antimicrobial composition. IgG in a pure form means that IgG is obtained by purification, or that IgG is synthesized chemically, or that IgG is synthesized after molecular engineering. The glycosylated IgG constitutes in the range of 0.1 to 90% by weight of the composition, such as at most 90% by weight of the composition, such as at most 80% by weight of the composition, such as at most 70% by weight of the composition, such as at most 60% by weight of the composition, such as at most 50% by weight of the composition, such as at most 40% by weight of the composition, such as at most 30% by weight of the composition, such as at most 20% by weight of the composition, such as at most 10% by weight of the composition, such as at most 9% by weight of the composition, such as at most 8% by weight of the composition, such as at most 7% by weight of the composition, such as at most 6% by weight of the composition, such as at most 5% by weight of the composition, such as at most 4% by weight of the composition, such as at most 3% by weight of the composition, such as at most 2% by weight of the composition, such as at most 1.5% by weight of the composition, such as at most 1% by weight of the composition, such as at most 0.75% by weight of the composition, such as at most 0.5% by weight of the composition, such as at most 0.4% by weight of the composition. In another preferred embodiment of the invention the amount of glycosylated IgG is at least 0.1 % by weight of the composition, such as at least 0.2% by weight of the composition, such as at least 0.3% by weight of the composition, such as at least 0.375% by weight of the composition, such as at least 0.4% by weight of the composition. Preferably the amount of glycosylated IgG is in the range of 0.4% to 1.2% by weight of the composition, in particular the amount of glycosylated IgG is 0.4% or 1.2% by weight of the composition.

In a further embodiment of the invention the glycosylated immunoglobulins are only of the IgM class, whereby extracted glycosylated IgM is used as the only immunoglobulin in the antimicrobial composition. Furthermore, glycosylated IgM in a pure form can be mixed with pooled immunoglobulins in the antimicrobial composition. IgM in a pure form means that IgM is obtained by purification, or that IgM is synthesized chemically, or that IgM is synthesized after molecular engineering. The glycosylated IgM constitutes in the range of 0.1 to 90% by weight of the composition, such as at most 90% by weight of the composition, such as at most 80% by weight of the composition, such as at most 70% by weight of the composition, such as at most 60% by weight of the composition, such as at most 50% by weight of the composition, such as at most 40% by weight of the composition, such as at most 30% by weight of the composition, such as at most 20% by weight of the composition, such as at most 10% by weight of the composition, such as at most 9% by weight of the composition, such as at most 8% by weight of the composition, such as at most 7% by weight of the composition, such as at most 6% by weight of the composition, such as at most 5% by weight of the composition, such as at most 4% by weight of the composition, such as at most 3% by weight of the composition, such as at most 2% by weight of the composition, such as at most 1.5% by weight of the composition, such as at most 1% by weight of the composition, such as at most 0.75% by weight of the composition, such as at most 0.5% by weight of the composition, such as at most 0.4% by weight of the composition. In another preferred embodiment of the invention the amount of glycosylated IgM is at least 0.1 % by weight of the composition, such as at least 0.2% by weight of the composition, such as at least 0.3% by weight of the composition, such as at least 0.375% by weight of the composition, such as at least 0.4% by weight of the composition. Preferably the amount of glycosylated IgM is in the range of 0.4% to 1.2% by weight of the composition, in particular the amount of glycosylated IgM is 0.4% or 1.2% by weight of the composition.

In yet a further embodiment of the invention the glycosylated immunoglobulins are only of the IgA class, whereby extracted glycosylated IgA is used as the only immunoglobulin in the antimicrobial composition. Furthermore, glycosylated IgA in a pure form can be mixed with pooled immunoglobulins in the antimicrobial composition. IgA in a pure form means that IgA is obtained by purification, or that IgA is synthesized chemically, or that IgA is synthesized after molecular engineering. The glycosylated IgA constitutes in the range of 0.1 to 90% by weight of the composition, such as at most 90% by weight of the composition, such as at most 80% by weight of the composition, such as at most 70% by weight of the composition, such as at most 60% by weight of the composition, such as at most 50% by weight of the composition, such as at most 40% by weight of the composition, such as at most 30% by weight of the composition, such as at most 20% by weight of the composition, such as at most 10% by weight of the composition, such as at most 9% by weight of the composition, such as at most 8% by weight of the composition, such as at most 7% by weight of the composition, such as at most 6% by weight of the composition, such as at most 5% by weight of the composition, such as at most 4% by weight of the composition, such as at most 3% by weight of the composition, such as at most 2% by weight of the composition, such as at most 1.5% by weight of the composition, such as at most 1 % by weight of the composition, such as at most 0.75% by weight of the composition, such as at most 0.5% by weight of the composition, such as at most 0.4% by weight of the composition. In another preferred embodiment of the invention the amount of glycosylated IgA is at least 0.1 % by weight of the composition, such as at least 0.2% by weight of the composition, such as at least 0.3% by weight of the composition, such as at least 0.375% by weight of the composition, such as at least 0.4% by weight of the composition. Preferably the amount of glycosylated IgA is in the range of 0.4% to 1.2% by weight of the composition, in particular the amount of glycosylated IgA is 0.4% or 1.2% by weight of the composition.

In yet another embodiment of the invention the glycosylated immunoglobulins are only of the dimer IgA class, whereby extracted glycosylated dimer IgA is used as the only immunoglobulin in the antimicrobial composition. Furthermore, glycosylated dimer IgA in a pure form can be mixed with pooled immunoglobulins in the antimicrobial composition. Dimer IgA in a pure form means that dimer IgA is obtained by purification, or that dimer IgA is synthesized chemically, or that dimer IgA is synthesized after molecular engineering. The glycosylated dimer IgA constitutes in the range of 0.1 to 90% by weight of the composition, such as at most 90% by weight of the composition, such as at most 80% by weight of the composition, such as at most 70% by weight of the composition, such as at most 60% by weight of the composition, such as at most 50% by weight of the composition, such as at most 40% by weight of the composition, such as at most 30% by weight of the composition, such as at most 20% by weight of the composition, such as at most 10% by weight of the composition, such as at most 9% by weight of the composition, such as at most 8% by weight of the composition, such as at most 7% by weight of the composition, such as at most 6% by weight of the composition, such as at most 5% by weight of the composition, such as at most 4% by weight of the composition, such as at most 3% by weight of the composition, such as at most 2% by weight of the composition, such as at most 1.5% by weight of the composition, such as at most 1 % by weight of the composition, such as at most 0.75% by weight of the composition, such as at most 0.5% by weight of the composition, such as at most 0.4% by weight of the composition. In another preferred embodiment of the invention the amount of glycosylated dimer IgA is at least 0.1% by weight of the composition, such as at least 0.2% by weight of the composition, such as at least 0.3% by weight of the composition, such as at least 0.375% by weight of the composition, such as at least 0.4% by weight of the composition. Preferably the amount of glycosylated dimer IgA is in the range of 0.4% to 1.2% by weight of the composition, in particular the amount of glycosylated dimer IgA is 0.4% or 1.2% by weight of the composition.

Another embodiment of the invention the glycosylated immunoglobulins are only of the IgY class, whereby extracted glycosylated IgY is used as the only immunoglobulin in the antimicrobial composition. Furthermore, glycosylated IgY a pure form can be mixed with pooled immunoglobulins in the antimicrobial composition. IgY in a pure form means that IgY is obtained by purification, or that IgY is synthesized chemically, or that IgY is synthesized after molecular engineering. The glycosylated IgY constitutes in the range of 0.1 to 90% by weight of the composition, such as at most 90% by weight of the composition, such as at most 80% by weight of the composition, such as at most 70% by weight of the composition, such as at most 60% by weight of the composition, such as at most 50% by weight of the composition, such as at most 40% by weight of the composition, such as at most 30% by weight of the composition, such as at most 20% by weight of the composition, such as at most 10% by weight of the composition, such as at most 9% by weight of the composition, such as at most 8% by weight of the composition, such as at most 7% by weight of the composition, such as at most 6% by weight of the composition, such as at most 5% by weight of the composition, such as at most 4% by weight of the composition, such as at most 3% by weight of the composition, such as at most 2% by weight of the composition, such as at most 1.5% by weight of the composition, such as at most 1 % by weight of the composition, such as at most 0.75% by weight of the composition, such as at most 0.5% by weight of the composition, such as at most 0.4% by weight of the composition. In another preferred embodiment of the invention the amount of glycosylated IgY is at least 0.1% by weight of the composition, such as at least 0.2% by weight of the composition, such as at least 0.3% by weight of the composition, such as at least 0.375% by weight of the composition, such as at least 0.4% by weight of the composition. Preferably the amount of glycosylated IgY is in the range of 0.4% to 1.2% by weight of the composition, in particular the amount of glycosylated IgY is 0.4% or 1.2% by weight of the composition.

Lysozyme is found in a number of biological fluids such as in saliva, egg white, tears, blood and milk. Lysozyme cleaves proteoglycans of the peptidoglycan layer of bacterial cell walls such as between N-acetylmuraminic acid and N-acetylglucosamine whereby the cell wall structure is broken, a process known as lysis or bacteriolysis.

In one embodiment of the invention lysozyme is extracted from biological fluids as described above, such as is extracted from hen's egg white. In a second embodiment of the invention lysozyme is obtained by genetic engineering as a recombinant protein.

Furthermore, the lysozyme of the antimicrobial composition may be native, whereby the term native means non-conjugated. In another embodiment of the invention the lysozyme is conjugated. In a particular embodiment the lysozyme may be conjugated to a monosaccharide to enable the binding of lysozyme to bacteria. A particularly preferred monosaccharide is mannose.

In a preferred embodiment of the invention the amount of lysozyme is in the range of 0.05 to 10% by weight of the composition, such as at most 10% by weight of the composition, such as at most 9% by weight of the composition, such as at most 8% by weight of the composition, such as at most 7% by weight of the composition, such as at most 6% by weight of the composition, such as at most 5% by weight of the composition, such as at most 4% by weight of the composition, such as at most 3% by weight of the composition, such as at most 2% by weight of the composition, such as at most 1.5% by weight of the composition, such as at most 1 % by weight of the composition, such as at most 0.75% by weight of the composition, such as at most 0.5% by weight of the composition. In another preferred embodiment of the invention the amount of lysozyme is at least 0.05% by weight of the composition, such as at least 0.1 % by weight of the composition, such as at least 0.2% by weight of the composition, such as at least 0.3% by weight of the composition, such as at least 0.4% by weight of the composition, such as at least 0.5% by weight of the composition. Preferably the amount of lysozyme is 0.5% by weight of the composition.

The composition according to the invention may be formulated into any suitable formulation. In one embodiment the antimicrobial composition is for local use on mucosal membranes. The formulation is selected from the groups of a lozenge, a tablet to be chewed, chewing gum, a cream, a gel, a wet tissue, a paste, a lotion, an ointment and a liniment. In particular, the formulation is a lozenge. Another preferred formulation is chewing gum. It is further preferred that the formulation is selected from the groups of a cream, a gel, a wet tissue, a paste, a lotion, an ointment and a liniment. In a particular preferred embodiment the formulation is selected form the group of a cream, a gel and a wet tissue.

In another embodiment the formulation is for consumption and is selected from the groups of breast milk substitute, food for human consumption and animal feed.

In yet another embodiment of the invention the antimicrobial composition is for local use on skin. Accordingly, for use on skin the formulation is selected from the groups of a cream, a gel, a wet tissue, a paste, a lotion, an ointment and a liniment. In a particular preferred embodiment the formulation is selected form the group of a cream, a gel and a wet tissue.

A second aspect of the invention relates to an antimicrobial composition, comprising lysozyme conjugated to a monosaccharide to enable the binding of lysozyme to bacteria. It is desirable that the lysozyme is conjugated to mannose.

Lysozyme is found in a number of biological fluids such as in saliva, egg white, tears, blood and milk. Lysozyme cleaves proteoglycans of the peptidoglycan layer of bacterial cell walls such as between N-acetylmuraminic acid and N-acetylglucosamine whereby the cell wall structure is broken, a process known as lysis or bacteriolysis.

In one embodiment of the invention lysozyme is extracted from biological fluids as described above. In a second embodiment of the invention lysozyme is obtained by genetic engineering as a recombinant protein.

In a preferred embodiment of the invention the amount of conjugated lysozyme is in the range of 0.05 to 10% by weight of the composition, such as at most 10% by weight of the composition, such as at most 9% by weight of the composition, such as at most 8% by weight of the composition, such as at most 7% by weight of the composition, such as at most 6% by weight of the composition, such as at most 5% by weight of the composition, such as at most 4% by weight of the composition, such as at most 3% by weight of the composition, such as at most 2% by weight of the composition, such as at most 1.5% by weight of the composition, such as at most 1 % by weight of the composition, such as at most 0.75% by weight of the composition, such as at most 0.5% by weight of the composition. In another preferred embodiment of the invention the amount of lysozyme is at least 0.05% by weight of the composition, such as at least 0.1% by weight of the composition, such as at least 0.2% by weight of the composition, such as at least 0.3% by weight of the composition, such as at least 0.4% by weight of the composition, such as at least 0.5% by weight of the composition. Preferably the amount of lysozyme is 0.5% by weight of the composition.

In one embodiment the antimicrobial composition is for local use on mucosal membranes. The formulation is selected from the groups of a lozenge, a tablet to be chewed, chewing gum, a cream, a gel, a wet tissue, a paste, a lotion, an ointment and a liniment. In particular, the formulation is a lozenge. Another preferred formulation is chewing gum. It is further preferred that the formulation is selected from the groups of a cream, a gel, a wet tissue, a paste, a lotion, an ointment and a liniment. In a particular preferred embodiment the formulation is selected form the group of a cream, a gel and a wet tissue.

In another embodiment the formulation is for consumption and is selected from the groups of breast milk substitute, food for human consumption and animal feed.

In yet another embodiment of the invention the antimicrobial composition is for local use on skin. Accordingly, for use on skin the formulation is selected from the groups of a cream, a gel, a wet tissue, a paste, a lotion, an ointment and a liniment. In a particular preferred embodiment the formulation is selected form the group of a cream, a gel and a wet tissue.

### Method for preparing the antimicrobial composition

The antimicrobial composition according to the invention may be prepared by any suitable method. Thus, a third aspect of the invention concerns a method of preparing the composition by mixing lysozyme as described above with the glycosylated immunoglobulins and subsequently mixing the lysozyme/immunoglobulin mixture with additives relevant for the formulation in question as discussed above. In another embodiment all ingredients are mixed simultaneously.

The glycosylation of the immunoglobulins may be carried out in any suitable manner. In one embodiment the immunoglobulins are dissolved in a solution comprising the disaccharide or monosaccharide in question. In a preferred embodiment the immunoglobulins are dissolved in a glucose solution and incubated for a predetermined period of time to allow the immunoglobulins to be glycosylated. Examples of other monosaccharides and disaccharides are discussed above.

In one embodiment the lysozyme is conjugated to another moiety. Thus, a fourth aspect of the invention concerns a method for preparing the antimicrobial composition comprising conjugated lysozyme, wherein the lysozyme is conjugated prior to the mixing with other ingredients, The other moiety is preferably a moiety capable of directing the lysozyme to the microbe in question. Thus, in one embodiment the lysozyme is conjugated to mannose.

### Formulation and dosage

In a fifth aspect of the invention the antimicrobial composition may be formulated in any suitable formulation depending on the localisation of the infection to be treated and/or prevented. The infections are caused by Gram negative bacteria.

The antimicrobial composition may be administered at least once daily, such as at least twice daily. Each dosage preferably comprises at least 0.1 mg lysozyme and at least 0.01 mg immunoglobulin, such as at least 0.5 mg lysozyme and at least 0.05 mg immunoglobulin, such as at least 1.0 mg lysozyme and at least 0.1 mg immunoglobulin.

### Use

In a sixth aspect the invention relates to the use of lysozymes and glycosylated immunoglobulins for the preparation of a antimicrobial composition as described above. In particular the invention relates to the use of lysozymes and glycosylated immunoglobulins for the preparation of a antimicrobial composition for preventing and/or treating an infection caused by Gram negative bacteria.

### Examples

Non classified Gram negative rods and Gram negative cocci were used in laboratory experiments. The used lysozyme is extracted from hen's egg white and the immunoglobulins have been isolated from bovine milk and colostrum.

### Example 1

### Experiment 1a

The suspension of bacteria (100.000 bacteria per ml) was incubated for half an hour in the presence of 5 mg per ml lysozyme at 37°C. Subsequent culturing on an agar plate did not show bacterial kill.

### Experiment 1b

The suspension of bacteria (100.000 bacteria per ml) was incubated for half an hour in the presence of 5 mg per ml lysozyme + 40 micrograms per ml agglutinating native antibodies at 37°C. Subsequent culturing on an agar plate did not show bacterial kill.

### Experiment 1c

The suspension of bacteria (100.000 bacteria per ml) was incubated for half an hour in the presence of 5 mg per ml lysozyme + 40 micrograms per ml agglutinating glycosylated antibodies at 37°C. Subsequent culturing on an agar plate showed 100 % bacterial kill.

### Example 2

### Experiment 2a

The suspension of bacteria consisting of 2 different types of Gram negative bacteria are incubated for half an hour at 37°C in the presence of 5 mg per ml lysozyme + 40 micrograms per ml agglutinating native antibodies specific for one of the bacteria types. Subsequent culturing on an agar plate did not show bacterial kill.

### Experiment 2b

The suspension of bacteria consisting of 2 different types of Gram negative bacteria are incubated for half an hour at 37°C in the presence of 5 mg per ml lysozyme + 40 micrograms per ml agglutinating glycosylated antibodies specific for one of the bacteria types. Subsequent culturing on an agar plate showed 100 % bacterial kill of the bacteria type to which the antibodies were directed against, and no effect on the bacteria that did not agglutinate with the used antibodies.

To correlate the in vitro experiments to the in vivo situation the suspension of bacteria was a growth medium such that the exogenous bacterial enzymes were present.

Examples of compositions are shown below. The percentages are all weight percentages.

### Example 3

### 1) Composition for skin infections and infections of mucosal membranes (gel)

| | |
|---|---|
| Lysozyme | 0.5% |
| Glycosylated immunoglobulins | 0.4% |
| (pooled IgM, IgG, IgA). | |
| Resingel | 99.1% |
| | 100.0% |

### Example 4

### 2) Composition for skin infections and infections of mucosal membranes (cream)

| | |
|---|---|
| Lysozyme | 0.5% |
| Glycosylated immunoglobulins | 0.4% |
| (pooled IgM, IgG, IgA) | |
| Cream base | 99.1% |
| | 100.0% |

### Example 5

### 3) Composition for skin infections and infections of mucosal membranes (wet tissue)

| | |
|---|---|
| Lysozyme | 0.5% |
| Glycosylated immunoglobulins | 0.4% |
| Oil of peppermint | 0.1% |
| Sterile water | 99.0% |
| | 100.0% |

### Example 6

### 3) Lozenge for infections of mouth, throat and pharynx

| | |
|---|---|
| Lysozyme | 0.5% |
| Glycosylated immunoglobulins | 0.4% |
| Flavour additives and tablet additives | 99.1% |
| | 100.0% |

### Example 7

### 4) Tablet to be chewed for infections of the gastrointestinal tract

| | |
|---|---|
| Lysozyme | 0.5% |
| Glycosylated immunoglobulins | 1.2% |
| Flavour additives and tablet additives | 98.3% |
| | 100.0% |

### Example 8

### Chewing gum with antimicrobial composition

| | |
|---|---|
| Lysozyme | 0.5% |
| Glycosylated immunoglobulins | 0.4% |
| Chewing gum | 99.1% |
| | 100.0% |

### Example 9

### Chewing gum base

| | |
|---|---|
| Gum base | 19.0% |
| Powder sugar | 61.4% |
| Glucose | 18.0% |
| Glycerine | 1.0% |
| Flavour | 0.6% |
| | 100.0% |

### Example 10

### Sugar-free chewing gum base

| | |
|---|---|
| Gum base | 32.0% |
| Sorbitol powder | 47.8% |
| Sorbitol 70% | 13.0% |
| Mannitol | 4.0% |
| Glycerine | 2.0% |
| Soya Lecithin | 0.2% |
| Flavour | 1.0% |
| | 100.0% |

### Example 11

### Composition for infections of mucosal membranes (vaginal gel)

| | |
|---|---|
| Hydroxyethyl cellulose | 2.0% |
| Lysozyme | 0.5% |
| Glycosylated immunoglobulins | 0.4% |
| Water | 97.1% |
| | 100.0% |

### Example 12

### Breast milk substitute

| | |
|---|---|
| Protein | 1.5% |
| Fat | 3.5% |
| Carbohydrates | 7.5% |
| Lysozyme | 0.5% |
| Glycosylated immunoglobulins | 0.6% |
| Vitamins | q.s |
| Minerals | q.s |
| Water | q.s |
| | 100.0% |

### Example 13

### Supplement for animal feed

| | |
|---|---|
| Lyophilised culture of bifido and acidophilus bacteria | 7.2% |
| Lysozyme | 0.8% |
| Glycosylated immunoglobulins | 1.0% |
| Glucose | 91.0% |
| | 100.0% |

## Claims

1. Antimicrobial composition comprising lysozyme and glycosylated immunoglobulins directed against antigens present on the surface of Gram negative bacteria, wherein said glycosylated immunoglobulins have been produced by being dissolved in a solution comprising disaccharide or monosaccharide.

2. Antimicrobial composition according to claim 1 for local use on mucosal membranes and/or skin.

3. Antimicrobial composition according to claim 1, wherein the Gram negative bacteria are rods and/or cocci or a combination thereof.

4. Antimicrobial composition according to claim 1, wherein the glycosylated immunoglobulins have affinity to antigen determinants on the cell wall of Gram negative bacteria.

5. Antimicrobial composition according to claim 1, wherein the glycosylated immunoglobulins are of monoclonal or polyclonal origin.

6. Antimicrobial composition according to claim 1, wherein the glycosylated immunoglobulins are of monoclonal and/or polyclonal origin or a combination thereof.

7. Antimicrobial composition according to claim 1, wherein the glycosylated immunoglobulins are of the classes IgM, IgG, IgY, IgA or dimer IgA.

8. Antimicrobial composition according to claim 1, wherein the glycosylated immunoglobulins are of the IgG class and/or the IgY class.

9. Antimicrobial composition according to any of the preceding claims, wherein the glycosylated immunoglobulins are intact and/or resistant to proteases such as bacterial proteases and/or pancreatic proteases.

10. Antimicrobial composition according to any of the claims 1 to 8, wherein the glycosylated immunoglobulins are intact and/or resistant to proteolytic enzymes such as papain and/or bromelain and/or pepsin.

11. Antimicrobial composition according to any of the claims 1 to 8, wherein the glycosylated immunoglobulins are intact and/or resistant to acidic conditions such as in gastric juice.

12. Antimicrobial composition according to any of the claims 1 to 8, wherein the glycosylated immunoglobulins have lost their ability of complement fixation.

13. Antimicrobial composition according to claim 1, wherein the glycosylated immunoglobulins originate from a biological fluid such as milk, whey, blood, plasma, colostrum, yolk or serum.

14. Antimicrobial composition according to claim 1, wherein the glycosylated immunoglobulins originate from a biological fluid such as milk and/or colostrum and/or yolk and/or a combination thereof.

15. Antimicrobial composition according to claim 1, wherein the glycosylated immunoglobulins originate from immunized animals and/or non-immunized animals.

16. Antimicrobial composition according to claim 1, wherein the lysozyme is native or conjugated.

17. Antimicrobial composition according to claim 16, wherein the lysoxyme is conjugated to a monosaccharide.

18. Antimicrobial composition according to claim 17, wherein the lysozyme is conjugated to mannose.

19. Antimicrobial composition according to claim 1, wherein the lysozyme is extracted from egg white.

20. Antimicrobial composition according to claim 1, wherein the antimicrobial composition is selected from the groups of a cream, an ointment, a gel, a wet tissue, a tablet to chew, a lozenge and chewing gum.

21. Antimicrobial composition according to claim 1, wherein the antimicrobial composition is in the form of a lozenge or chewing gum.

22. Antimicrobial composition according to claim 1, wherein said lysozyme constitutes in the range of 0.05% to 10% by weight of the composition.

23. Antimicrobial composition according to claim 1, wherein said glycosylated immunoglobulins constitute in the range of 0.1% to 10% by weight of the composition.

24. A method for the preparation of the antimicrobial composition according to any of the claims 1-23 comprising the steps of
a) using immunoglobulins
b) glycosylating the immunoglobulins
c) obtaining native or conjugated lysozyme
d) mixing the glycosylated immunoglobulins and the lysozyme, and optionally adding additives, thereby obtaining the antimicrobial composition.

25. Use of lysozymes and glycosylated immunoglobulins for the preparation of an antimicrobial composition as defined by any of the claims 1-23.

26. Use of lysozymes and glycosylated immunoglobulins for the preparation of an antimicrobial composition as defined by any of the claims 1-23 for the prophylaxis and/or treatment of an infection.

27. A composition as defined by any of the claims 1-23 for use as a medicament.

## Patentansprüche

1. Antimikrobielle Zusammensetzung, die Lysozym und glycosylierte Immunoglobuline, die gegen Antigene gerichtet sind, die auf der Oberfläche von gram-negativen Bakterien vorhanden sind, umfasst, wobei die glycosylierten Immunoglobuline durch ein Auflösen in einer Disaccharid oder Monosaccharid umfassenden Lösung gebildet wurden.

2. Antimikrobielle Zusammensetzung nach Anspruch 1 zur lokalen Verwendung auf Schleimhautmembranen und/oder Haut.

3. Antimikrobielle Zusammensetzung nach Anspruch 1, wobei die gram-negativen Bakterien Stäbchen und/oder Kokken oder eine Kombination hiervon sind.

4. Antimikrobielle Zusammensetzung nach Anspruch 1, wobei die glycosylierten Immunoglobuline eine Affinität zu Antigendeterminanten auf der Zellwand von gram-negativen Bakterien aufweisen.

5. Antimikrobielle Zusammensetzung nach Anspruch 1, wobei die glycosylierten Immunoglobuline von monoklonalem oder polyklonalem Ursprung sind.

6. Antimikrobielle Zusammensetzung nach Anspruch 1, wobei die glycosylierten Immunoglobuline von monoklonalem und/oder polyklonalem Ursprung oder einer Kombination hiervon sind.

7. Antimikrobielle Zusammensetzung nach Anspruch 1, wobei die glycosylierten Immunoglobuline aus den Klassen IgM, IgG, IgY, IgA oder Dimer-lgA stammen.

8. Antimikrobielle Zusammensetzung nach Anspruch 1, wobei die glycosylierten Immunoglobuline aus der lgG-Klasse und/oder der lgY-Klasse stammen.

9. Antimikrobielle Zusammensetzung nach einem der vorhergehenden Ansprüche, wobei die glycosylierten Immunoglobuline intakt und/oder gegenüber Proteasen, wie Bakterienproteasen und/oder Pankreasproteasen, resistent sind.

10. Antimikrobielle Zusammensetzung nach einem der Ansprüche 1 bis 8, wobei die glycosylierten Immunoglobuline intakt und/oder gegenüber proteolytischen Enzymen, wie Papain und/oder Bromelain und/oder Pepsin, resistent sind.

11. Antimikrobielle Zusammensetzung nach einem der Ansprüche 1 bis 8, wobei die glycosylierten Immunoglobuline intakt und/oder gegenüber sauren Bedingungen, wie in Magensaft, resistent sind.

12. Antimikrobielle Zusammensetzung nach einem der Ansprüche 1 bis 8, wobei die glycosylierten Immunoglobuline ihre Fähigkeit zur Komplementfixierung verloren haben.

13. Antimikrobielle Zusammensetzung nach Anspruch 1, wobei die glycosylierten Immunoglobuline aus einem biologischen Fluidum, wie Milch, Molke, Blut, Plasma, Kolostrum, Eigelb oder Serum herrühren.

14. Antimikrobielle Zusammensetzung nach Anspruch 1, wobei die glycosylierten Immunoglobuline aus einem biologischen Fluidum, wie Milch und/oder Kolostrum und/oder Eigelb und/oder einer Kombination hiervon herrühren.

15. Antimikrobielle Zusammensetzung nach Anspruch 1, wobei die glycosylierten Immunoglobuline aus immunisierten Tieren und/oder nicht immunisierten Tieren herrühren.

16. Antimikrobielle Zusammensetzung nach Anspruch 1, wobei das Lysozym nativ oder konjugiert ist.

17. Antimikrobielle Zusammensetzung nach Anspruch 16, wobei das Lysozym an ein Monosaccharid konjugiert ist.

18. Antimikrobielle Zusammensetzung nach Anspruch 17, wobei das Lysozym an Mannose konjugiert ist.

19. Antimikrobielle Zusammensetzung nach Anspruch 1, wobei das Lysozym aus Eiweiß extrahiert ist.

20. Antimikrobielle Zusammensetzung nach Anspruch 1, wobei die antimikrobielle Zusammensetzung aus den Gruppen einer Creme, einer Salbe, eines Gels, eines feuchten Tuchs, einer Kautablette, einer Pastille und eines Kaugummis ausgewählt ist.

21. Antimikrobielle Zusammensetzung nach Anspruch 1, wobei die antimikrobielle Zusammensetzung in Form einer Pastille oder eines Kaugummis vorliegt.

22. Antimikrobielle Zusammensetzung nach Anspruch 1, wobei das Lysozym einen Bereich von 0,05 bis 10 Gew.-% der Zusammensetzung bildet.

23. Antimikrobielle Zusammensetzung nach Anspruch 1, wobei die glycosylierten Immunoglobuline einen Bereich von 0,1 bis 10 Gew.-% der Zusammensetzung bilden.

24. Verfahren zur Herstellung der antimikrobiellen Zusammensetzung nach einem der Ansprüche 1 bis 23, das die folgenden Stufen umfasst:
a) Verwenden von Immunoglobulinen
b) Glycosylieren der Immunoglobuline
c) Erhalten von nativem oder konjugiertem Lysozym
d) Mischen der glycosylierten Immunoglobuline und des Lysozyms und optionales Zugeben von Additiven, wodurch die antimikrobielle Zusammensetzung erhalten wird.

25. Verwendung von Lysozymen und glycosylierten Immunoglobulinen zur Herstellung einer antimikrobiellen Zusammensetzung gemäß Definition in einem der Ansprüche 1 bis 23.

26. Verwendung von Lysozymen und glycosylierten Immunoglobulinen zur Herstellung einer antimikrobiellen Zusammensetzung gemäß Definition in einem der Ansprüche 1 bis 23 zur Prophylaxe und/oder Behandlung einer Infektion.

27. Zusammensetzung gemäß Definition in einem der Ansprüche 1 bis 23 zur Verwendung als Medikament.

## Revendications

1. Composition antimicrobienne comprenant du lysozyme et des immunoglobulines glycosylées dirigées contre des antigènes présents à la surface de bactéries Gram négatif, où lesdites immunoglobulines glycosylées ont été produites par dissolution dans une solution comprenant un disaccharide ou un monosaccharide.

2. Composition antimicrobienne selon la revendication 1, en vue d'une utilisation locale sur des muqueuses et/ou la peau.

3. Composition antimicrobienne selon la revendication 1, où les bactéries Gram négatif sont des bâtonnets et/ou des cocci ou une combinaison de ceux-ci.

4. Composition antimicrobienne selon la revendication 1, dans laquelle les immunoglobulines glycosylées présentent une affinité envers des déterminants antigéniques sur la paroi cellulaire des bactéries Gram négatif.

5. Composition antimicrobienne selon la revendication 1, dans laquelle les immunoglobulines glycosylées sont d'origine monoclonale ou polyclonale.

6. Composition antimicrobienne selon la revendication 1, dans laquelle les immunoglobulines glycosylées sont d'origine monoclonale et/ou polyclonale ou une combinaison de celles-ci.

7. Composition antimicrobienne selon la revendication 1, dans laquelle les immunoglobulines glycosylées sont des classes des IgM, IgG, IgY, IgA ou un dimère d'IgA.

8. Composition antimicrobienne selon la revendication 1, dans laquelle les immunoglobulines glycosylées sont de la classe des IgG et/ou de la classe des IgY.

9. Composition antimicrobienne selon l'une quelconque des revendications précédentes, dans laquelle les immunoglobulines glycosylées sont intactes et/ou résistantes à des protéases telles que des protéases bactériennes et/ou des protéases pancréatiques.

10. Composition antimicrobienne selon l'une quelconque des revendications 1 à 8, dans laquelle les immunoglobulines glycosylées sont intactes et/ou résistantes à des enzymes protéolytiques telles que la papaïne et/ou la bromélaïne et/ou la pepsine.

11. Composition antimicrobienne selon l'une quelconque des revendications 1 à 8, dans laquelle les immunoglobulines glycosylées sont intactes et/ou résistantes à des conditions acides telles dans le suc gastrique.

12. Composition antimicrobienne selon l'une quelconque des revendications 1 à 8, dans laquelle les immunoglobulines glycosylées ont perdu leur capacité de fixation du complément.

13. Composition antimicrobienne selon la revendication 1, dans laquelle les immunoglobulines glycosylées proviennent d'un liquide biologique tel que le lait, le lactosérum, le sang, le plasma, le colostrum, le vitellus ou le sérum.

14. Composition antimicrobienne selon la revendication 1, dans laquelle les immunoglobulines glycosylées proviennent d'un liquide biologique tel que le lait et/ou le colostrum et/ou le vitellus et/ou une combinaison de ceux-ci.

15. Composition antimicrobienne selon la revendication 1, dans laquelle les immunoglobulines glycosylées proviennent d'animaux immunisés et/ou d'animaux non immunisés.

16. Composition antimicrobienne selon la revendication 1, dans laquelle le lysozyme est natif ou conjugué.

17. Composition antimicrobienne selon la revendication 16, dans laquelle le lysozyme est conjugué à un monosaccharide.

18. Composition antimicrobienne selon la revendication 17, dans laquelle le lysozyme est conjugué à du mannose.

19. Composition antimicrobienne selon la revendication 1, dans laquelle le lysozyme est extrait du blanc d'oeuf.

20. Composition antimicrobienne selon la revendication 1, où la composition antimicrobienne est choisie dans le groupe d'une crème, d'une pommade, d'un gel, d'une lingette, d'un comprimé à mâcher, d'une pastille et d'un chewing-gum.

21. Composition antimicrobienne selon la revendication 1, où la composition antimicrobienne est sous la forme d'une pastille ou d'un chewing-gum.

22. Composition antimicrobienne selon la revendication 1, dans laquelle ledit lysozyme se situe dans la plage de 0,05 % à 10 % en poids de la composition.

23. Composition antimicrobienne selon la revendication 1, dans laquelle lesdites immunoglobulines glycosylées se situent dans la plage de 0,1 % à 10 % en poids de la composition.

24. Procédé de préparation de la composition antimicrobienne selon l'une quelconque des revendications 1 à 23 comprenant les étapes consistant à
a) utiliser des immunoglobulines
b) glycosyler les immunoglobulines
c) obtenir du lysozyme natif ou conjugué
d) mélanger les immunoglobulines glycosylées et le lysozyme, et éventuellement à ajouter des additifs, obtenant de cette manière la composition antimicrobienne.

25. Utilisation de lysozymes et d'immunoglobulines glycosylées pour la préparation d'une composition antimicrobienne telle que définie par l'une quelconque des revendications 1 à 23.

26. Utilisation de lysozymes et d'immunoglobulines glycosylées pour la préparation d'une composition antimicrobienne telle que définie par l'une quelconque des revendications 1 à 23 destinée à la prophylaxie et/ou au traitement d'une infection.

27. Composition telle que définie par l'une quelconque des revendications 1 à 23 en vue d'une utilisation en tant que médicament.
